Europäisches Patentamt

European Patent Office

Office européen des brevets

(11) Publication number: **0 351 891 B1**

# EUROPEAN PATENT SPECIFICATION

(45) Date of publication of patent specification: **29.09.93**   (51) Int. Cl.5: **G01N 27/30**, C12M 1/40

(21) Application number: **89116797.5**

(22) Date of filing: **08.05.84**

(60) Publication number of the earlier application in accordance with Art.76 EPC: **0 127 958**

(54) **Printed electrodes.**

(30) Priority: **05.05.83 GB 8312262**
**05.05.83 GB 8312261**
**06.09.83 GB 8323799**
**16.12.83 GB 8333644**
**11.01.84 GB 8400650**
**29.02.84 GB 8405262**
**29.02.84 GB 8405263**

(43) Date of publication of application:
**24.01.90 Bulletin  90/04**

(45) Publication of the grant of the patent:
**29.09.93 Bulletin  93/39**

(84) Designated Contracting States:
**BE CH DE FR GB IT LI NL SE**

(56) References cited:
**EP-A- 0 030 503      EP-A- 0 078 636**
**EP-A- 0 152 541      DE-A- 2 127 142**
**US-A- 4 185 131      US-A- 4 224 125**
**US-A- 4 229 490      US-A- 4 376 689**

(73) Proprietor: **MediSense, Inc.**
**266 Second Avenue**
**Waltham Massachusetts 02154(US)**

(72) Inventor: **Hill, Hugh Allen Oliver**

**Nine Clover Close**
**Oxford OX2 9JH(GB)**
Inventor: **Higgins, Irving John**
**Cotswold Graze Hill**
**Ravensden Bedford MK44 2TF(GB)**
Inventor: **McCann, James Michael**
**33 Dewhurst Road**
**West Kensington London W14 0ES(GB)**
Inventor: **Davis, Graham**
**1504 Fox Run Drive**
**Plainsboro NJ 08536(US)**
Inventor: **Treidl, Bernhard Ludwig**
**15528 Grinnel Terrace**
**Derwood Station MD 20855(US)**
Inventor: **Birket, Nigel Norman**
**Drayton House Station Road**
**Bloxham Banbury Oxon(GB)**
Inventor: **Plotkin, Elliot Verne**
**14 George Street**
**Bedford MK4 3SG(GB)**
Inventor: **Zwanziger, Ron**
**322 Waverley Avenue**
**Newton,MA 02158(US)**

(74) Representative: **Ruffles, Graham Keith et al**
**MARKS & CLERK 57-60 Lincoln's Inn Fields**
**London WC2A 3LS (GB)**

## Description

THIS INVENTION relates to the fabrication of electrodes, and in particular to the fabrication of electrodes by printing. More especially, the present invention relates to a suspension formed as a printable and conductive ink for use in the fabrication of electrodes.

Our European Patent Application 82305597 (EP-A 78636) describes the construction of sensors comprising a conductive electrode coated with a mixture, or layers, of a catalytically active enzyme and a mediator compound and usually further coated with a retaining permeable membrane. When such a coated electrode is contacted with a substrate containing a species for which the enzyme exerts a catalytic effect, the mediator compound transfers charge to the electrode and this can be used to give a readout signal, against a standard electrode, correlated with the concentration of the said species, even in the presence of other species since enzymes are typically highly selective in their catalytic action.

Enzymes which have been investigated and utilised in such systems are listed elsewhere in this specification.

Thus, numerous types of enzyme-coated electrodes have been utilised each specific to the presence of a physiological or other substrate for which the particular enzyme acts as a catalyst and each therefore potentially capable of acting to detect, measure or monitor the level of the substrate in vivo or in vitro and give a readout correlated for instance with an underlying physiological condition controlling or affecting the substrate level. In particular, use of glucose oxidase or bacterial glucose dehydrogenase as the enzyme, associated with suitable electron-transferring mediator compounds, has been shown to give readout signals correlating linearly with in vitro blood glucose levels over a wide range thus giving a diagnostic or measuring tool for diabetic conditions. Also, such electrodes can measure glucose levels in plasma, serum, interstital fluid, saliva or urine.

The mediator compounds described in our EP-A 78636 include polyviologens, fluoranil and chloranil. However, the preferred mediator compounds are metallocene compounds, and in particular the ferrocenes (biscyclopentadienyl iron and its derivatives).

The particular advantages of ferrocenes are as follows:-
(a) a wide range of redox potentials accessible through substitution of the cylopentadienyl rings (b) functionalisation of the rings, e.g. to confer solubility or chemical linkability to other such rings or other system components (c) electrochemically reversible one-electron redox properties (d) pH -independent redox potential and (e) slow autooxidation of the reduced form.

The ferrocene structure may be modified by substitution on the rings, and/or by association or polymerisation, which modifications affect the physical, chemical and electrical behaviour so that optimisation of a particular sensor electrode material is possible. In general use, the compound 1,1'dimethylferrocene is a valuable mediator.

In general, the equipment disclosed in EP-A 78636 is suitable for research or institutional (e.g. hospital) use. The present invention is concerned with providing sensor electrodes and equipment for use by lay persons or without extensive technical back-up services.

Use may be made of such electrodes in chemical industry especially where complex mixtures are encountered, e.g. in food chemistry or biochemical engineering. They are however of particular value in biological investigation or control techniques, in human or animal medicine.

We have now established certain design criteria in the production of such electrodes for lay, or clinic, use.

The electrodes can be used in an invasive probe (i.e. one which enters body tissue to contact a body fluid such as whole blood or subcutaneous tissue fluid) or as part of an external test upon a withdrawn sample (using a syringe) or upon an expressed sample (e.g. using a needle-prick device). In each instance the electrode must be as small as practical to avoid trauma either on invasion of the tissue or withdrawing of the sample. If invasive, it must be throwaway, to avoid cross-contamination and to prevent encapsulation by fibroblast cells which occurs with long-term implants.

It must be elongate, either to fit within a pointed needle, or for ready handling as an electrode for ready assembly to equipment on the one hand and contact with the sample on the other. It must be sensitively manipulable. It must carry, prior to assembly or in the assembled structure, the reference electrode as well as the 'sensitive' electrode, in spaced non-contiguous relationship.

Derived from these design criteria, sensor electrode systems form the subject of our European Patent EP-127958 B1 (European Patent Application 84303091.7, from which the present application was divided).

The present invention is directed at the fabrication of electrodes, and in particular at the fabrication of electrodes by printing.

EP 0 351 891 B1

European Patent specification EP-152541-A published 28 August 1985 describes the manufacture of an amperometric electrode based on enzyme catalysis of a reaction of a substrate present in solution. Working and reference electrodes are formed as thin deposits on an inert base of sintered ceramic oxide by screen printing, using inks respectively comprising on the one hand a platinum powder and a glass powder base, and on the other hand a silver powder and a glass powder base. The deposits are then fired at elevated temperature. The sintered ceramic oxide base is porous or a glass or porous vitroceramic membrane is formed on the working electrode. An activated silane is grafted onto the porous base or the porous membrane, and at least one enzyme is then grafted onto the silane. Grafting of the enzyme is typically achieved by immersion of the porous silanized membrane in a solution of the enzyme for 24 hours at 4°C.

German Offenlegungsschrift 2127142 describes the fabrication of analytical devices comprising an electrically insulating base member, a pair of spaced electrically conductive thin film electrode elements carried on the base, and an overlying semi-permeable matrix containing a test reagent. Typically, metal film is deposited by thermal evaporation onto a ceramic base and then etched using photoresist printing techniques to form the spaced electrode elements. The matrix with enzyme as test reagent is then applied by dip coating.

US Patent 4224125 describes an enzyme electrode in which an oxidoreductase enzyme and a redox compound acting as mediator are immobilized on or in the neighbourhood of an electrode. Various methods are described for immobilization of the enzyme and/or mediator, including covalent bonding of the mediator to the electrode, immobilization of the enzyme by cross-linking, and physical entrapment of the mediator and enzyme as a gel in a defined space adjacent the electrode. In this last instance, the mediator is in the form of a redox polymer, rendering it insoluble, and Example 2 discloses the inclusion of carbon powder in the gel.

In accordance with the present invention, there is provided a suspension formed as a printable and conductive ink for use in the fabrication of electrodes, the suspension comprising a liquid medium containing (a) carbon, together with at least one of (b) an enzyme and (c) a mediator compound.

Both an enzyme and a mediator can be present in the ink.

In a preferred aspect, the enzyme is glucose oxidase and the mediator is ferrocene or a ferrocene derivative.

The present invention further provides for the use by printing of the suspensions of this invention, in the fabrication of an electrode.

The present invention also provides a method of manufacturing a sensor electrode system which is generally elongate and thereby manipulable, the sensor electrode system comprising an electrode carrier in the form of a flat strip carrying a working electrode and a reference electrode in spaced non-contiguous relationship, in which method an enzyme and/or a mediator are applied to the flat strip by printing a suspension of the present invention.

The suspensions provided by the present invention can be used for example in the manufacture of electrode sensor systems for selective detection, measurement or monitoring of a given dissolved substrate in a mixture of dissolved substrates, comprising:-

(a) an area of first electrode material comprising an enzyme catalytic of the said substrate and a mediator compound to transfer charge to the electrode when the enzyme is catalytically active, adjacent to but non-contiguous with;

(b) an area of reference electrode material; both electrodes being of small dimension, and extending as or supported on an elongate member to facilitate manipulation before or during contact with live tissue or a small withdrawn sample of body fluid.

The sensor electrodes can be dipped into or similarly contacted with a liquid substrate e.g. a glucose-containing small blood sample or drop of blood.

In one aspect, a sensor can be manufactured for contact with a liquid mixture of components for detecting the presence of, measuring the amount of and/or monitoring the level of one or more selected components capable of undergoing an enzyme catalysed reaction, the sensor comprising:-

(a) an elongate support member,

(b) on a surface thereof towards one end an expanse of a first electrode of electrically conductive material comprising at least at an outer surface thereof the combination of an enzyme and a mediator compound which transfers electrons to the first electrode when the enzyme is catalytically active,

(c) on a surface of the elongate support member and also towards the said end thereof to be in close proximity therewith an expanse of a second, reference, electrode, and

(d) separate electrical connection to each electrode for attachment to a read-out means denoting presence, amount, or monitored level of the said one or more selected components in a liquid medium into which the support member is dipped to contact both electrodes.

3

The elongate support conveniently comprises a flat strip.

The first electrode is preferably formed of carbon e.g. a filter paper containing carbon. We have also found that carbon foil e.g. as available under the Trade Marks "GRAPHOIL" or "PAPYEX" is a valuable electrode material. The enzyme thereon can in theory be any enzyme, e.g. those listed in our European Application 84303090.9 but the use of glucose oxidase or dehydrogenase, e.g. the bacterial glucose dehydrogenase from Acinetobacter calcoaceticus is particularly valuable. Any suitable mediator compound can be used, but ferrocene or ferrocene derivatives (especially 1,1'-dimethylferrocene) are greatly to be preferred.

The second electrode can be any convenient reference electrode. We have found it useful to provide adjacent but not contiguous to the first electrode, a flat layer of silver and to convert the surface thereof to silver chloride so as to give an Ag/AgCl reference electrode.

Typically, the electrical connections can be wires which extend down, and are preferably adhered to, the strip, and make electrical contact each with its respective electrode.

The readout means is preferably a digital indicator suitably connected to a dedicated potentiostat which poises the carbon electrode potential at e.g. +150mV Ag/AgCl for a glucose system. The current flowing is then proportional to glucose concentration.

In a particularly valuable version of this type of sensor, it comprises (a) a flat first electrode area of known area small enough to be completely coverable by the smear of blood produced from a non-expressed drop of blood generated from a needle-prick at a bodily extremity, (b) a reference electrode area on the same surface separate from but sufficiently close to the sensitive electrode area that the said blood smear also reaches the reference electrode to establish electrical communication and (c) conductive elements extending separately along the same surface of,and thus insulated from the elongate support member, communicating one with each electrode for connection to signal read-out means attachable to one end of the member.

The area of the first (i.e. sensitive) electrode is generally substantially square; it may be rectangular or otherwise shaped, but in any case usually will correspond in area to a square of 5 mm edge length, or below e.g. from 2 to 4 mm.

Equipment utilising the sensors can be portable or "desktop".

In one form, equipment utilises such electrodes to give visible readout correlated with a selected physiological parameter thus being capable of use in human or veterinary medicine by medical or nursing personnel, or by experienced lay subjects on a self-measurement basis.

For convenience, this document will refer hereinafter to blood-glucose-measuring equipment as being typical but not limitative of equipment which can employ electrodes fabricated using the printable suspensions of the present invention.

Diabetic subjects need to measure their glucose levels frequently. Hitherto, a common method carried out by the subject personally is a colorimetric test using a blood or urine sample which is applied over a surface area containing a colour-reactive detector chemical, adjacent to a comparison area, to give a colour change which is compared with a chart of colour values as an approximate measure of glucose level.

There are however defects in this method. Firstly, colorimetric changes are quantitatively difficult to assess, especially if the patient has impaired vision as a result of the diabetic condition. Indeed, because of this problem expensive automatic colour comparison equipment may need to be purchased by some subjects for interpreting the test results. Secondly, the blood test, while inherently more accurate than a urine test, needs a large enough sample to cover the test surface. Thirdly, it requires the patient to time the colour development accurately. Since blood samples, on a self-treatment basis are taken from body extremities (fingers, toes, earlobes) they are normally not large enough when obtained by a simple needle-prick, and must in fact be expressed i.e. squeezed or massaged out to form a larger drop. Progressively, the tissue of the extremities becomes scarred and coarsened by such treatment to an extent whereby finding fresh testing sites presents a problem.

For a home-diagnostic basis, a main object is the provision of small scale non-traumatic test pieces, either as a small diameter invasive probe electrode or as an external test electrode strip capable of using the naturally-arising small blood droplet from a needle-prick tester, without tissue massage. Examples are described in more detail below.

These small-scale electrodes are intended as single-use throwaway articles and are utilised in conjunction with electrical circuitry and a readout means, to which they must be easily attachable and detachable. Such circuitry and readout means is itself preferably embodied on a very small scale.

We have accordingly found that the totality of the equipment is subject to certain design constraints.

Thus the device should be nontraumatic to the user either physically e.g. if used with its own invasive probe or psychologically by virtue of its appearance. Further, the device should be capable, despite the small size of the throwaway electrode and of the permanent circuitry/readout components, of easy assembly and disassembly even by juvenile or elderly lay users. The relatively expensive permanent circuitry/readout components should, despite their small size, be of a form which minimizes loss or damage. The device should display readings which are visible and understandable to a non-expert user.

We have now found that these and other objects can be met by assembly of the circuitry/readout components into a housing resembling a pen or digital-watch.

Such an assembly of circuitry and display means for use in producing a readout value as a diagnostic aid in human or veterinary medicine, is housed in a pen-like hollow elongate housing having (a) at one end an electrically conductive socket suitable to receive the outer end of at least one detachable test member capable of producing an electrical signal correlating with a physiological parameter to which the test member is selectively sensitive and (b) towards the other end a digital read-out window for exhibiting a numerical value corresponding to the parameter. A thermister may also be used for temperature compensation.

The pen-like assembly as defined above can be used in combination with an attached test member, and there can be provided a kit of interrelated parts of such an assembly with a plurality of test members suitable for one-off use.

The term "pen-like" is a general limitation on size and shape. In functional terms, its characteristics are such that it can be held near the socket between the thumb and the nearer one or two opposed fingers, with the elongate body resting on and extending beyond the forefinger, but not to an extent that prejudices fine control of the socket end by the thumb and fingers. In numerical terms it can be from 10 to 30 cm. long and from 0.5 to 3 cms across its maximum transverse dimension; more usually it will be from 12 to 20 cms. long and from 0.8 to 1.5 cms. across. It can be generally circular, or polygonal, in cross-section. Each detachable test member is usually a small-scale enzyme-coated sensor electrode, of the type discussed in the earlier Patent Applications listed above, and especially such an electrode where the enzyme is specifically glucose-catalyzing whereby diabetic conditions can be measured. It may be a flat external strip electrode dimensioned to operate on a small, non-expressed, blood droplet. The socket arrangement will vary accordingly.

Two or more sensor electrodes may be incorporated into a single test member. Again, the socket arrangement will vary accordingly.

The readout means will typically be a conventional seven-segment display window towards the rearward end of the "pen" as in conventional pen/watches. In the case of the multiple sensor embodiment described in the preceding paragraph the display may be switchable between each sensor's discrete monitoring circuit, both the display and a single monitoring circuit may be switchable between sensors, or, a specific display may be provided for each of the sensors present.

Such equipment is of course particularly adapted for use with the non-invasive strip sensor defined above.

Another form of equipment for use with the sensor electrode systems is so-called "desk-top" equipment, i.e. for general but skilled use in a general clinic.

In this aspect, the aim is to provide suitable equipment for a practitioner of "desk-top" scale and complexity and while use of such equipment may be envisaged for any enzyme-catalysable component (with suitable electrode systems) for convenience this specification will refer to glucose determination as typical.

In the operation of a glucose sensor a number of relevant technical points and advantages should be considered. It will be appreciated that these points are of general relevance and should by no means be restricted in this application to particular design features: in other words, they apply generally to the embodiments listed above, and especially to those embodiments insofar as they deal with equipment and methods for glucose sensing. These features are:-

I Constructional Features

(a) Membrane cover for electrode

Although the enzyme electrode should be in electrical contact with the liquid, it may be found valuable to exclude the sensor from interfering contact with larger molecules or tissue fluid components. This can be done by a covering or surrounding membrane, depending on electrode geometry. Heat-shrinkable thin polymer tubing can be used as, or in connection with, such membranes.

The membranes can be polymerised in situ (e.g. cellulose acetate). A particular valuable membrane is formed by polycarbonate, especially those polycarbonates sold under the Trade Marks "NUCLEOPORE" or "STERILIN". When tissue fluids are examined they may contain ascorbate; polycarbonate membranes do not permit the passage of ascorbate and thus virtually eliminate interference from that substance. Alternatively a polyurethane membrane may be employed.

(b) Type of carbon

Carbon foil, as strips, or carbon attached to metal meshes, of pyrolytic grade and known by the Trade Marks "GRAPHOIL" and "PAPYEX" are much preferred for carbon-ferrocene electrodes for use with glucose oxidase. Oxygen interference is minimal, there being less than 4% change in signal between anaerobic and fully aerobic samples. Their physical nature is also very convenient for fabrication, especially of small-scale devices.

II Operational features

(a) Operational potential

Preferably operation should take place at a potential equivalent to +50 to +200 mV vs. SCE since interference caused by oxidation of other chemical species present is thereby reduced.

(b) Concentration range

Glucose oxidase can be used to monitor glucose concentrations of 0 to 40 mM, and glucose dehydrogenase at 0 to 20 mM when immobilised on a carbon-ferrocene electrode. The sensor response is linear up to about 40 mM.

(c) Response times

The glucose oxidase sensor without membrane is kinetically limited giving rapid response times i.e. about 20 seconds to 95% of the steady-state current response.

(d) Oxygen-sensitivity

Glucose dehydrogenase/ferrocene electrodes are totally oxygen-insensitive.

(e) Use of third electrode

In practice, a realistic device can achieve good performance without a third electrode, using Ag/AgCl as a reference counter-electrode, as described more fully below.

(f) pH and temperature

Glucose oxidase electrodes show no change in current output between pH6 and pH9, and are thus relatively pH-insensitive. They are temperature-stable up to 40ºC. If necessary temperature compensation can be effected using a thermistor, or a constant temperature jacket may be used. Also, operating with the electrodes diffusion-limited minimises temperature effects.

(g) Storage of Electrodes

Electrodes may be stored moist. Extended storage, over months or years, may be achieved by freeze-drying or air-drying.

Finally, some aspects can be considered of the electrical circuitry for operating the equipment as described.

The electrode sensor systems can be employed with a measuring device comprising means for comparing an electrical output of the electrode system with an electronic reference and means for providing a signal related to the electrical output of the electrode.

By employing an electronic reference rather than a cell or reference electrode a measurement using a sensor including an electron-transfer electrode may be made without the use of a separate electrode as a reference.

In a preferred embodiment of the invention, the electron-transfer electrode is poised at a fixed potential against a reference electrode, and the current flowing in the electron-transfer electrode is measured.

The particular electron transfer electrode may be selected from a range of electrodes including those employing the following enzymes

| Enzyme | Substrate |
|---|---|
| Flavo-proteins | |
| Pyruvate Oxidase | Pyruvate |
| L-Amino Acid Oxidase | L-Amino Acids |
| Aldehyde Oxidase | Aldehydes |
| Xanthine Oxidase | Xanthines |
| Glucose Oxidase | Glucose |
| Glycollate Oxidase | Glycollate |
| Sarcosine Oxidase | Sarcosine |
| Lactate Oxidase | Lactate |
| Glutathione Reductase | NAD(P)H |
| Lipoamide Dehydrogenase | NADH |
| PQQ Enzymes | |
| Glucose Dehydrogenase | Glucose |
| Methanol Dehydrogenase | Methanol and other Alkanols |
| Methylamine Dehydrogenase | Methylamine |
| Haem-Containing Enzymes | |
| Lactate Dehydrogenase (Yeast Cytochrome $b_2$) | Lactate |
| Horse-radish Peroxidase | Hydrogen Peroxide |
| Yeast Cytochrome c Peroxidase | Hydrogen Peroxide |
| Metalloflavoproteins | |
| Carbonmonoxide Oxidoreductase | Carbon Monoxide |
| Cuproproteins | |
| Galactose Oxidase | Galactose |

7

The invention will be further described with reference to the accompanying drawings, which includes a description of prototype products which were not fabricated using the printable suspension forming the subject of the present invention. In the drawings:-

Figure 1 is a front view of a strip-supported electrode configuration;

Figure 2 is a back view of the combination shown in Figure 1;

Figure 3 shows an alternative strip-supported electrode;

Figure 4 shows a strip-supported electrode which is a variant of Figure 3;

Figure 5 shows a modified connection of the strip electrode of Figures 3 and 4;

Figure 6 shows a further alternative supported electrode;

Figure 7 shows a combination of two electrode supports;

Figures 8a and 8b are general diagrammatic side views of a pen-like portable holder, of particular utility for the electrodes shown in Figures 3, 4 and 5, having an assembly of circuitry and having a read-out window;

Figure 9 shows a schematic diagram of one form of electrical circuitry for use with the electrodes and equipment of the present invention;

Figure 10 shows a more elaborated circuit diagram for use in the embodiment of Figure 9;

Figure 11 shows a schematic diagram of an alternative embodiment of electrical circuitry; and

Figure 12 shows the more elaborated circuit diagram of a yet further embodiment of circuitry.

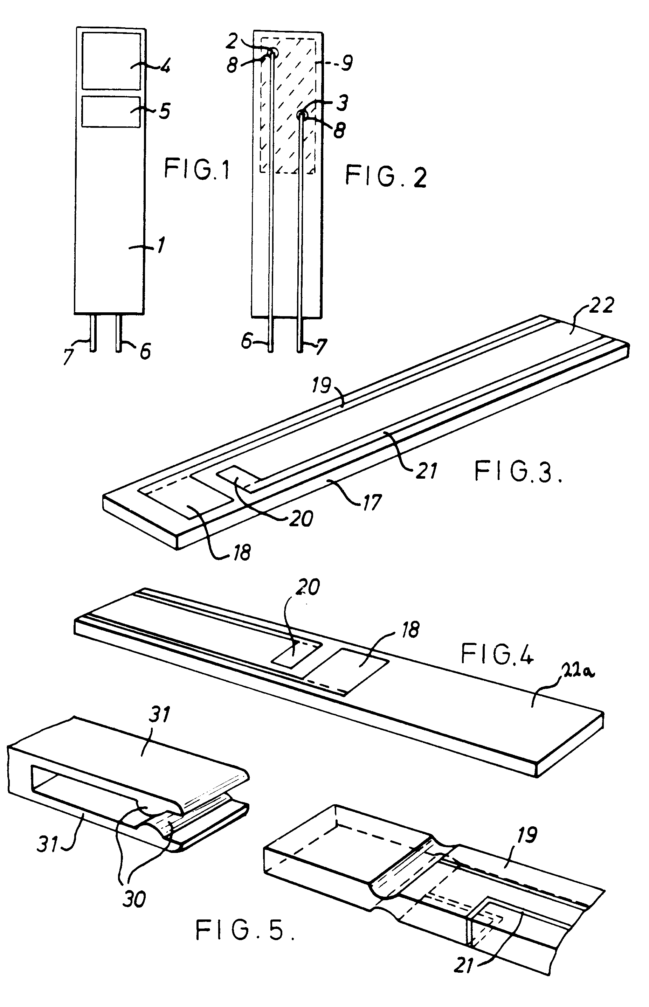

In the following description of Figures 1 and 2 dimensions, materials amount and proportions are given by way of example only.

A strip of epoxy glass 1, 9.5 x 40 x 1.6 mm, has two 1 mm diameter holes 2 and 3 therein. A 9 x 9 mm piece of graphite tape or foil 4 is glued on one face, near the end to cover hole 2 and a 4 x 9 mm strip of silver foil 5 is glued adjacent thereto over hole 3. Wires 6 and 7 (Fig. 2) on the back enter holes 2 and 3 respectively for electrical connection with the respective electrode material 4 and 5, being glued in the holes by conductive epoxy resin 8. A stabilising layer of epoxy resin is present over at least part of the back e.g. at 9 to keep the wires in place. Carbon electrode 4 is covered with 1, 1'-dimethylferrocene and glucose oxidase. Silver electrode 5 is covered with silver chloride.

The strip is made up in the following sequence:-

(a) drill holes 2 and 3,

(b) glue on electrodes 4 and 5; "ARALDITE" epoxy resin is suitable but should not enter holes 2 and 3,

(c) attached wires 6 and 7, using conductive epoxy 8, and apply "ARALDITE" resin at 9 the fix the wires in place,

(d) hold the silver electrode at +400 mV vs. SCE in 5M chloride for 10-15 seconds to deposit a thin AgCl layer,

(e) apply a solution of 1,1'-dimethylferrocene (4 $\mu$l) in toluene (20 mg/ml) to the graphite tape 4 and allow to evaporate,

(f) cover the ferrocene-coated tape with 50 $\mu$l of carbodiimide (25 mg/ml) in pH 4.5 acetate buffer for 1 1/2 hours and

(g) rinse and cover with glucose oxidase (12.5 mg/ml) in pH 5.5 acetate buffer for 2 hours.

The strip can be used by attaching wires 6 and 7 to a potentiostat poising the potential at electrode 4 at +150 mV. vs. Ag/AgCl, and dipping the strip into a glucose-containing solution so that both electrodes 4 and 5 are covered. The shape of the strip facilitates such handling. The current flowing is proportional to glucose concentration.

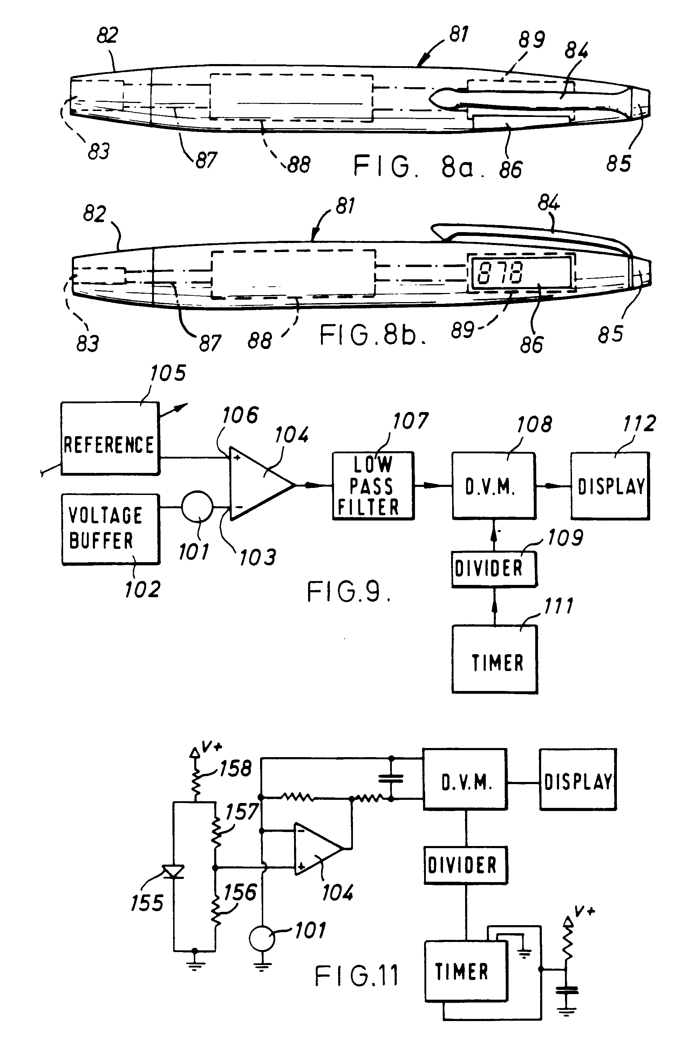

Figure 3 shows a strip electrode 17 made of, for example, a ceramic material or printed-circuit-board laminate. It includes a square area 18 with connector lead 19, the square being covered with the enzyme-containing layers as described above. It further includes a small reference electrode area 20 and separate connector lead 21. The rearward end 22 of the strip electrode 17 fits into a socket as shown in Figures 8a and 8b and described below. It is to be noted that the electrode strip 17 is a small-scale device. Thus square area 18 is of a side length only about half that of each of two square colorimetric test areas of conventional diagnostic tests and can be used with the original non-expressed bead of blood from a needle-prick device, which is adequate to cover the whole of the square area and communicates electrically with reference electrode area 20.

Modifications may be made to the embodiments shown in Figure 3. For example the strip electrode as shown in Figure 3 can be longer, whereby electrodes 18 and 20 are located only partway along the strip, leaving a free end 22a to facilitate ease of handling by the patient without damaging or touching the electrodes. Also, the electrode strip can clip within two opposed contacts or resilient mounting 31, the routing of one or other of conductive lines 19 and 21 being modified accordingly.

8

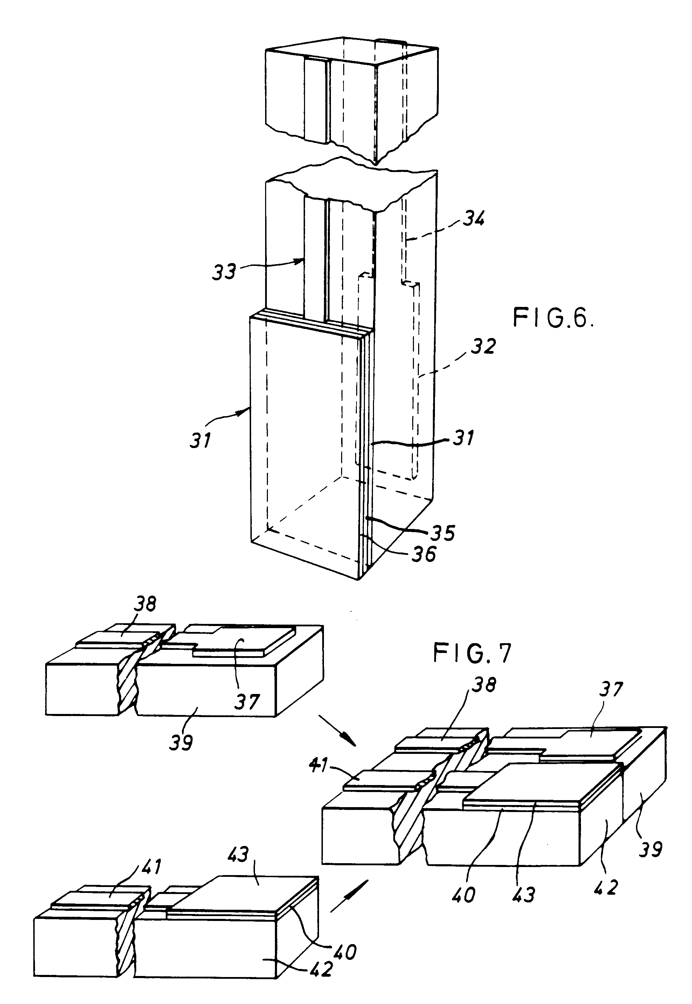

Figure 4 shows a longer strip, and Figure 5 shows the inner end of a strip held between two resilient metal contact strips.

In Figure 6 a 2 cm length of electrically insulating polymer for example MYLAR or TEFLON ( a polyfluorocarbon) 0.3 mm square in transverse cross-section is provided with a palladium-silver conductive electrode 31, on the front surface as shown, and a second, smaller electrode 32, on the back as shown in dotted lines. In each case conductive lines 33 and 34 respectively, were formed simultaneously with the electrodes.

On the front electrode 31 is painted a mixture of toluene, 1,1'-dimethyl ferrocene and graphite, formed by mixing a solution of the toluene and 1,1'-dimethylferrocene and a slurry of toluene and graphite. It is believed that the ferrocene is adsorbed on to graphite particles. After drying the mixture forms a layer 35. A layer 36 of glucose oxidase is then immobilised on the graphite surface by carbodiimide immobilisation, known per se (enzyme adsorption can also be used). The electrode may then be covered, on both sides, with a semipermeable membrane of cellulose acetate (or polyurethane), not shown, to block large interfering species from contact with the electrode.

The square section of the support helps in the painting of slurry, or the enzyme-attachment stages, in keeping the electrodes 31 and 32 distinct.

The small scale electrode so produced could be used per se but is especially valuable for incorporation into a standard gauge needle, giving a blood-glucose reading using the same invasive member as the eventual injection.

When producing such small scale needles, the exact sequence of steps can vary. For example, graphite could first be painted on, and a solution of the mediator (ferrocene, etc) in toluene then be applied by dipping into the graphite. Likewise, the enzyme can be applied in solution for adsorption. There is therefore a danger that the reference electrode e.g. silver/palladium could be adversely affected by the solvents or solutes used.

Figure 7 shows the key steps of a procedure which can be used to advantage in the fabrication of these microelectrodes.

The reference electrode 37 and its conductive lead-out strips 38 are formed in silver/palladium on TEFLON base 39. Similarly, an electrode support 40 and lead-out strip 41 are formed in silver/palladium on TEFLON base 42. Only this base 42 and its electrode support are then subjected to (a) painting on a graphite slurry in toluene (b) dipping in 1,1'dimethylferrocene solution in toluene and (c) contacting with the enzyme to absorb e.g. glucose oxidase into the active layer 43. Thereafter the bases 39 and 42 are glued or held, side-by-side, their general rectangular cross-section facilitating such positive location. Back-to back location is also possible.

As before, the finished assembly may be located inside a needle bore, e.g. with extra access portions near the electrode surfaces.

Incorporation of 1,1' dimethylferrocene into the electrode

A solution of 1,1'-dimethylferrocene in toluene was mixed into a toluene-based slurry of the graphite powder. The mixture was then painted on to the base conductor 41 and allowed to dry at 43. This provided an electrode surface that was electroactive towards glucose oxidase.

These experiments showed that "thick layer"or screen-printing technology could provide a usable base strip which could easily be coated with a stable graphite surface and that moreover the electrode surface could be made electro-active towards glucose by adsorption of a ferrocene directly into the coating mixture. In addition, the reference electrode operated satisfactorily in buffered solutions.

Figures 8a and 8b show a holder which is particularly adapted to utilise electrodes as shown in Figures 3, 4, 5 but which could if necessary utilise electrodes as shown in Figures 1 and 2, and 6 and 7 at least of the various embodiments shown.

From above the holder 81 intentionally resembles a conventional pen/watch as much as possible. It has a forward end 82, possibly rotary to tighten the walls of a flattened socket cavity 83 formed within it. A central join, a clip 84 and a press-button 85 all resemble those of a conventional pen, and digital readout-window 86 is also of a type known in pen/watches.

Inside the holder as shown by dotted lines is connection circuitry 87, possibly printed in situ, battery 88 and operating circuitry 89 behind and manufactured as a unit with the display window 86. The display can be capable of operation only when button 85 is pressed so that extra illumination can be provided if necessary.

The embodiments shown in Figures 8a and 8b especially when used in conjunction with the electrodes of Figures 3 - 5 fulfill the design criteria discussed above for such portable equipment.

The delicate manipulation facilitated by the pengrip (e.g. by thumb and finger) means that the small electrodes e.g. of Figures 3, 4 or 5 can be easily assembled into, or detached from, the socket. A user will always orient the holder with the window 86 visible thus always giving a uniform relative orientation to the socket 83 whereby the rearward ends of the fragile electrodes can be fitted without experiment and damage.

The "pen" format is instinctively picked up after use and safely carried in a pocket, more so than for any other small device. Thus the expensive part of the equipment is safeguarded. Furthermore, it is possible to incorporate a conventional timer circuit into the device thereby fulfilling the actual function of a pen-type watch and providing an audible or visible signal which marks the point in time at which a reading should be taken.

Finally, the display is numerical, clearly visible and if necessary can be supplemented by an illuminating light source.

In Figure 9, a sensor 101 is connected between a voltage buffer 102, and the inverting input 103 of the operational amplifier 104 which is configured as a current amplifer. An electronic reference 105 connected to the non-inverting input 106 of the operational amplifier is fed into a low-pass filter 107 which removes rapid signal fluctuations (which may be due to noise, earth hum or other sources of interference) while allowing the filtered output of the operational amplifier 104 to be fed into the digital volt meter (D.V.M.) 108.

The digital volt meter is supplied with clock pulses via the divider 109, from the timer 111. The D.V.M drives a liquid crystal display 112. The electronic reference 105, is either of a pre-selected value or capable of being selected for a particular electron transfer electrode.

In each of the embodiments of electrode discussed above the sensor comprises a mediator-carrying surface which has one or more enzymes immobilized thereupon. The sensor further includes a silver/silver chloride (Ag/AgCl) internal reference electrode. If, for example, a voltage of $+200$ mV volts is preferentially dropped across the electrode as is the case with a glucose -oxidase-containing electrode, then the reference voltage 105 is selected accordingly.

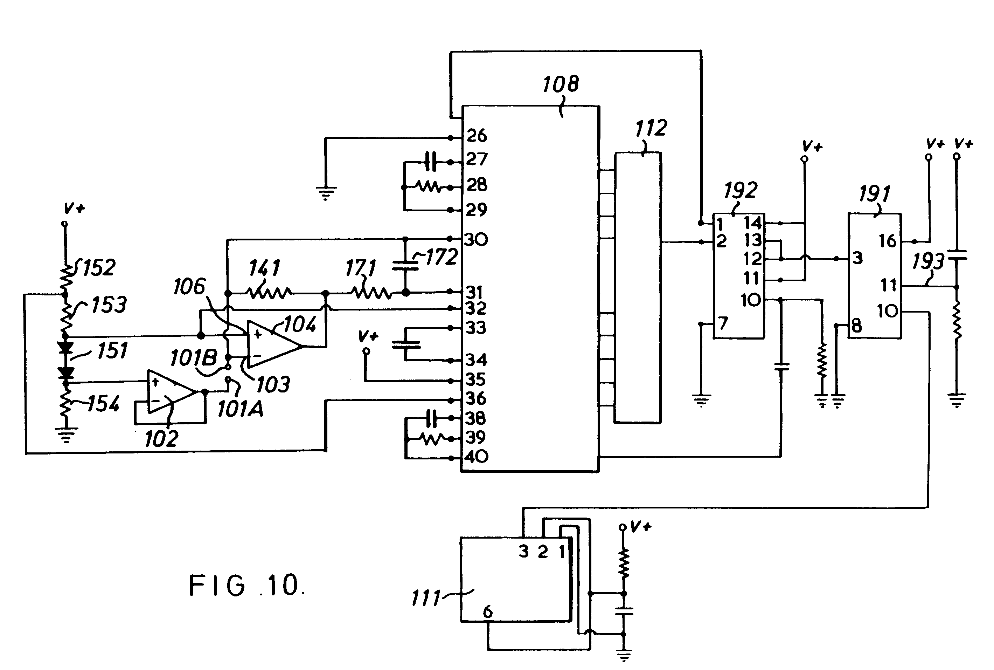

In Figure 10, the electronic reference comprises resistors 152, 153 and 154 together with diodes 151. A voltage is selected at 0.3V by a suitable choice of resistor values at 152, 153 and 154. The voltage across the resistor 153 is 1V, which ensures that 100 $\mu$A of electrode current will give a full scale reading of 999 on the liquid crystal display 112.

Non-inverting buffer 102 ensures that the voltage on the terminal 101A remains substantially constant The sensor 1 (Figure 1) is connected across terminals 101A and 101B.

The operational amplifier 104 is connected via its inverting input 103 to the terminal 101B and the feedback resistor 141. The non-inverting input 106 is connected to the electronic reference.

A first-order low-pass filter 171, 172 is connected across the feed-back resistor 141, and supplies an analog signal to the D.V.M. 108. Pin values are given for a 7116CPL chip (manufactured by Motorola). The D.V.M. drives a liquid crystal display 112.

Clock pulses for the D.V.M. are supplied from the timer 111, (pin values are given for a 555 chip) via the dividers 191 (pin values for an MC 14020B) and 192 (pin values for an MC14016B chip). The connection 193 to pin 11 of the divider 191 enables a power-up reset.

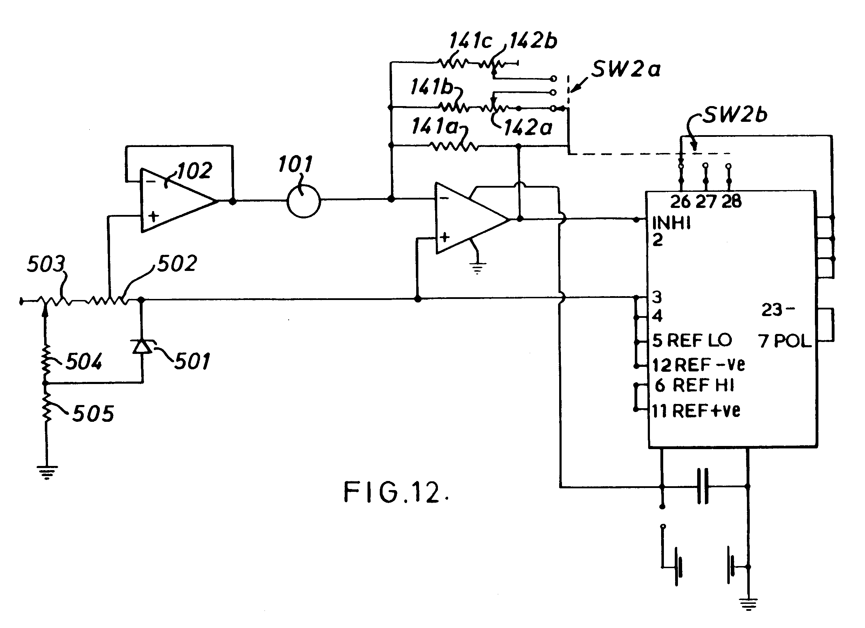

In Figure 11 an alternative circuit is shown which does not make use of the non-inverting voltage buffer 102, but has the sensor 101 connected between the operational amplifier 104, and ground. The embodiment shown in Figure 11 employs a fixed reference voltage which is provided by a circuit differing from that of Figure 10.

In this embodiment, the diode 155 functions as a voltage-reference diode and provides a reference voltage drop across its ends equal to the diode forward voltage.

By a suitable choice of the values of the resistors 156, 157 and 158 the correct voltage may be applied across the electrode. In this embodiment the sensor again employs as a reference an Ag/AgCl couple and immobilized glucose oxidase in the presence of a mediator compound as the electron-transfer electrode.

Figure 12 shows a third embodiment of the present invention, which provides a continuously variable reference voltage which may be selected to accommodate any type of electron-transfer electrode, that is, one, which for example, employs any of the enzymes listed herein or any combination of these enzymes. The LED display is not shown.

In Figure 12 the feedback resistor 141 in circuit at any given time may be selected from resistors 141a, 141b, and 141c by means of switch SW2a which is ganged with switch SW2b. This allows the current output of the sensor 101 to be displayed in three ranges, for example, 1 $\mu$A, 10 $\mu$A or 100 $\mu$A full scale. Furthermore, the range may be trimmed by using the variable resistors 142a and 142b.

The embodiment shown in Figure 12 has the non-inverting voltage buffer 102 of the embodiment shown in Figure 10.

The reference voltage for the embodiment shown in Figure 12, is derived from the circuit elements 501-505 which include the potentiometers 503 and 502 providing a variable voltage across the sensor 101, thereby accommodating any type of electron-transfer electrode. It is envisaged that the continuously variable resistors 142a and 142b could be replaced in certain applications by stepwise resistance switching means with each position or setting being dedicated to a particular type of electrode.

Various modifications may be made in the circuitry. For example the liquid crystal display may be replaced by a plotter or a dosage control device, or a temperature stability circuit may be incorporated.

Various modifications may also be made in constructional techniques for the electrode manufacture.

The electrode may for example be manufactured by screen printing techniques e.g. in a multi-stepped procedure comprising:-

I - screen printing of Ag/AgCl reference electrode and metal tracing.

II - screen printing of the active electrode with a printing ink comprising a colloidal carbon, glucose oxidase in buffer, and an organic polymer.

III - screen printing, spraying or dip coating to provide a membrane over the assembly.

Advantages of this method are that it is amenable to high volume automation, and is of high reproducibility.

From the above it follows that a suspension in a liquid medium of carbon together with at least one of (a) an enzyme and (b) a mediator compound capable of transferring charge to the said carbon from the enzyme when the enzyme is catalytically active can be used to fabricate electrodes, provided that the said suspension is formed as a printable and conductive ink. Preferably, both the enzyme (e.g. glucose oxidase) and the mediator (e.g. ferrocene or a ferrocene derivative) are present in the ink formulation.

Copending Application EP 84303090.9 of even date herewith entitled "Assay techniques utilising specific binding agents" is concerned with the effect on the enzyme and/or mediator electrochemical availability of specific binding agents e.g. antigens/antibodies and others. It can be embodied by specialised electrodes. Such electrodes can be fabricated using the present invention. The interested reader is referred to the published specification, EP-A-125139.

Thus, the mediator may be in the form of a mediator/hapten conjugate, i.e. be linked to a ligand material so that its activity in its charge-transferring property is a measure of further or competitive binding reaction with a specific binding agent with which the eventual electrode, having the specialised ink thereon, is contacted. A specific example is the theophylline/ferrocene conjugate described in copending Application entitled "Assay systems utilizing specific binding agents", of even date herewith. Other mediator/enzyme/ligand systems can also be utilized in the specialized ink.

More generally in this respect, the area of first electrode material may comprise (a) a ligand such as an antibody (b) an antiligand, such as a hapten , with specific binding properties thereto (c)the mediator, conjugated to either (a) or (b) and electrochemically active only when (a) and (b) are not specifically bound. Such an electrode is useful in assay of a system which unbinds (a) and (b), at least in part, thereby to provide mediator for the enzyme/substrate reaction.

## Claims

1. A suspension formed as a printable and conductive ink for use in the fabrication of electrodes, the suspension comprising a liquid medium containing (a) carbon, together with at least one of (b) an enzyme and (c) a mediator compound.

2. A suspension as claimed in claim 1 in which both an enzyme and a mediator are present.

3. A suspension as claimed in claim 1 or 2, in which the enzyme is glucose oxidase and the mediator is ferrocene or a ferrocene derivative.

4. The use by printing of a suspension as claimed in any preceding claim, in the fabrication of an electrode.

5. A method of manufacturing a sensor electrode system which is generally elongate and thereby manipulable, the sensor electrode system comprising an electrode carrier in the form of a flat strip carrying a working electrode and a reference electrode in spaced non-contiguous relationship, in which method an enzyme and/or a mediator are applied to the flat strip by printing a suspension as defined in any of claims 1 to 3.

**EP 0 351 891 B1**

**6.** A method according to claim 5, wherein the printing is performed by screen printing.

**Patentansprüche**

**1.** Eine Suspension, die als druckfähige und leitende Tinte ausgebildet ist zur Verwendung in der Fertigung von Elektroden, wobei die Suspension ein flüssiges Medium umfaßt, welches enthält: (a) Kohlenstoff, zusammen mit mindestens einem der folgenden: (b) ein Enzym und (c) eine Mittlerverbindung.

**2.** Eine Suspension nach Anspruch 1, in welcher ein Enzym und ein Mittler zusammen vorhanden sind.

**3.** Eine Suspension nach Anspruch 1 oder 2, in welcher das Enzym Glukose Oxidase ist und der Mittler Ferrocen oder ein Ferrocenabkömmling ist.

**4.** Verwendung der Suspension zum Drucken gemäß einer der vorangegangenen Ansprüche bei der Fertigung einer Elektrode.

**5.** Verfahren zur Herstellung eines Sensor-Elektrodensystems, welches üblicherweise länglich ist und damit handhabbar, wobei das Sensor-Elektrodensystem einen Elektrodenträger in der Form eines Streifens umfaßt, welcher eine Arbeitselektrode und eine Referenzelektrode in räumlich nicht zusammenhängender Beziehung trägt, bei welchem Verfahren ein Enzym und/oder ein Mittler an den flachen Streifen durch Drucken einer Suspension aufgebracht wird, wie in einem der Ansprüche 1 bis 3 definiert wurde.

**6.** Verfahren nach Anspruch 5, bei dem das Drucken durch Siebdruck bewerkstelligt wird.

**Revendications**

**1.** Suspension façonnée comme encre imprimable et conductrice pour une utilisation dans la fabrication d'électrodes, la suspension comprenant un milieu liquide contenant (a) du carbone en même temps que au moins un des (b) enzyme et (c) composé médiateur.

**2.** Suspension telle que revendiquée dans la revendication 1 dans laquelle à la fois une enzyme et un médiateur sont présents.

**3.** Suspension telle que revendiquée dans la revendication 1 ou 2, dans laquelle l'enzyme est la glucose oxydase et le médiateur est le ferrocène ou un dérivé du ferrocène.

**4.** Utilisation par impression d'une suspension telle que revendiquée dans l'une quelconque des revendications précédentes, dans la fabrication d'une électrode.

**5.** Procédé de fabrication d'un système d'électrodes pour détecteurs qui est, de manière générale, de forme allongée et de ce fait manipulable, le système d'électrodes pour détecteurs comprenant un support d'électrodes sous la forme d'une languette plate portant une électrode de travail et une électrode de référence dans une configuration spatiale non-contiguë, dans lequel procédé une enzyme et/ou un médiateur est (sont) appliqué(s) sur la languette plate par l'impression d'une suspension telle que définie dans l'une quelconque des revendications 1 à 3.

**6.** Procédé selon la revendication 5, dans laquelle l'impression est réalisée par impression au tamis.

12

FIG.1

FIG.2

FIG.3.

FIG.4

FIG.5.

FIG.6.

FIG.7

FIG. 8a.

FIG.8b.

FIG.9.

FIG.11

FIG .10.

EP 0 351 891 B1

FIG.12.

EP 0 351 891 B1